# EUROPEAN PATENT APPLICATION

(11) **EP 4 844 355 A1**
(43) Date of publication of application: **25.11.2026**
(21) Application number: 24921835.5
(22) Date of filing: 31.12.2024
(51) Int. Cl.: G01L 1/14, G01L 9/12, A61B 5/0245, C08G 75/045

(54) **SENSOR, DETECTION APPARATUS, WEARABLE DEVICE, AND IONIC GEL AND PREPARATION METHOD**

(30) Priority: 29.01.2024 CN 202410127044
(71) Applicant: Huawei Technologies Co., Ltd., Shenzhen, Guangdong 518129 (CN)
(72) Inventor: DING, Li, Shenzhen, Guangdong 518129 (CN); LIAO, Zelong, Shenzhen, Guangdong 518055 (CN); SHI, Junli, Shenzhen, Guangdong 518055 (CN); CHEN, Qing, Shenzhen, Guangdong 518129 (CN); YANG, Han, Shenzhen, Guangdong 518129 (CN); LIU, Chuangchuang, Shenzhen, Guangdong 518129 (CN)
(74) Representative: Huawei European IPR
(86) International application number: PCT/CN2024/144368
(87) International publication number: WO 2025/161813

(57) **Abstract**

This application provides a sensor, a detection apparatus, a wearable device, an ionic gel, and a preparation method thereof, and pertain to the field of sensor technologies. The sensor includes a support layer, a substrate layer, an electrode layer, an ionic gel layer, and a packaging layer. The electrode layer is disposed between the ionic gel layer and the substrate layer, the ionic gel layer is disposed between the electrode layer and the packaging layer, and the packaging layer is connected to the substrate layer through the support layer. The sensor is configured to receive a pressing force, so that a contact area between the ionic gel layer and the electrode layer changes. The sensor provided in embodiments of this application can detect static and dynamic forces.

## Description

This application claims priority to Chinese Patent Application No. 202410127044.4, filed with the China National Intellectual Property Administration on January 29, 2024 and entitled "SENSOR, DETECTION APPARATUS, WEARABLE DEVICE, AND IONIC GEL AND PREPARATION METHOD", which is incorporated herein by reference in its entirety.

### TECHNICAL FIELD

Embodiments of this application relate to the field of sensor technologies, and in particular, to a sensor, a detection apparatus, a wearable device, an ionic gel, and a preparation method.

### BACKGROUND

Currently, wearable devices, such as smart bands and smartwatches, integrate functions such as blood pressure measurement and heart rate measurement. A wearable device includes a pressurization mechanism and a piezoelectric pressure sensor. During a process in which the pressurization mechanism applies pressure to a wrist of a subject, the piezoelectric pressure sensor can detect changes in a pulse signal at the wrist. However, existing piezoelectric pressure sensors cannot detect a static force. Performance of sensors of the wearable devices needs to be improved.

### SUMMARY

Embodiments of this application provide a sensor, a detection apparatus, a wearable device, an ionic gel, and a preparation method. The sensor can detect static and dynamic forces. This helps improve performance of the wearable device.

A first aspect of this application provides a sensor, including a support layer, a substrate layer, an electrode layer, an ionic gel layer, and a packaging layer. The electrode layer is disposed between the ionic gel layer and the substrate layer, the ionic gel layer is disposed between the electrode layer and the packaging layer, and the packaging layer is connected to the substrate layer through the support layer. The sensor is configured to receive a pressing force, so that a contact area between the ionic gel layer and the electrode layer changes.

When the pressing force is a static force, the ionic gel layer comes into contact with the electrode layer, and an ion-electron pair is formed at a contact interface between the ionic gel layer and the electrode layer, so that the sensor can output a capacitance value, thereby implementing force detection. When the pressing force is a dynamic force, the contact area between the ionic gel layer and the electrode layer changes with the pressing, so that a quantity of ion-electron pairs formed at the contact interface between the ionic gel layer and the electrode layer changes, and as a result, the capacitance value output by the sensor changes, thereby implementing force detection. Therefore, by using the sensor provided in embodiments of this application to detect static and dynamic forces, the wearable device may eliminate the need for a dedicated pressure sensor for detecting a static force. In addition, this helps improve performance of the wearable device.

In addition, compared with a conventional parallel-plate capacitive force sensor, a change in the capacitance value output by the sensor provided in this application depends on a change in the quantity of ion-electron pairs at a contact interface between an ionic gel and an electrode. In addition, because a spacing between ion-electron pairs is far less than a thickness of a dielectric layer in a conventional parallel-plate capacitor, the capacitance value output by the sensor provided in this application is several orders of magnitude higher than that of the parallel-plate capacitor, increasing from a pF level to an nF level. This also improves sensitivity and force resolution of the sensor by 10 to 100 times.

In a possible implementation, the ionic gel layer and the electrode layer are disposed at intervals in a first direction, and the first direction is a thickness direction of the substrate layer.

In this way, when the sensor is pressed, the contact area between the ionic gel layer and the electrode layer may be expanded, and the quantity of ion-electron pairs may be increased, so that the capacitance value output by the sensor is increased, thereby helping improve sensitivity and force resolution of the sensor.

In a possible implementation, a microstructure is disposed on a surface of the ionic gel layer facing the electrode layer.

In this way, when the sensor is pressed, the contact area between the ionic gel layer and the electrode layer may be expanded, and the quantity of ion-electron pairs may be increased, so that the capacitance value output by the sensor is increased, thereby helping improve sensitivity and force resolution of the sensor. In addition, the ionic gel layer may be in contact with the electrode layer when the sensor is not pressed, thereby helping further reduce a size of the sensor in a thickness direction of a substrate.

In a possible implementation, the ionic gel layer includes 1,4-butanediol divinyl ether, 2,2'-(ethylenedioxy)diethanethiol, trimethylolpropane tris(3-mercaptopropionate), benzoin dimethyl ether, and 1-ethyl-3-methylimidazolium bis(trifluoromethylsulfonyl)imide.

In this way, the ionic gel layer is made of these materials, so that a quantity of ion-electron pairs formed when the ionic gel layer is in contact with the electrode layer can be increased, and sensitivity and force resolution of the sensor can be further improved.

In a possible implementation, the ionic gel layer is connected to the packaging layer through chemical bonding.

In this way, connection between the ionic gel layer and the packaging layer may be implemented by using a chemical connection method, so that firmness of the connection between the ionic gel layer and the packaging layer can be improved. In addition, a thickness of a connection structure between the ionic gel layer and the packaging layer may be further reduced, to reduce a thickness size of the sensor, and implement a thinning design.

In a possible implementation, the sensor further includes a coupling agent layer, the coupling agent layer is disposed between the ionic gel layer and the packaging layer, the ionic gel layer is connected to the coupling agent layer through chemical bonding, and the packaging layer is connected to the coupling agent layer through chemical bonding.

In this way, the ionic gel layer and the packaging layer can be firmly connected, and the thickness size of the sensor can be reduced, thereby facilitating a thinning design.

In a possible implementation, the support layer is provided with a through hole, and the ionic gel layer and the electrode layer are disposed inside the through hole.

In this way, the support layer can protect the electrode layer and the ionic gel layer, and the ionic gel layer can come into contact with the electrode layer when the sensor is pressed, to form an ion-electron pair.

In a possible implementation, the support layer includes a plurality of through holes disposed at intervals, and one ionic gel layer and one electrode layer are disposed in each of the through holes.

In this way, a plurality of ionic gel layers are separated from each other and a plurality of electrode layers are separated from each other through the support layer of a grid structure, so that an electrode layer and an ionic gel layer in each through hole form one matrix point, and the support layer may further separate adjacent matrix points. An ionic gel layer-electrode layer combination in each matrix point can independently output one group of capacitance values, and does not affect capacitance values of an ionic gel layer-electrode layer combination in an adjacent matrix point. Therefore, there is no crosstalk between matrix points, thereby ensuring measurement accuracy.

In a possible implementation, the packaging layer includes a first elastic layer and a second elastic layer, and the second elastic layer is disposed between the first elastic layer and the ionic gel layer and is bonded to the ionic gel layer. An elastic modulus of the first elastic layer is greater than or equal to 1 GPa and an elastic modulus of the second elastic layer is less than or equal to 100 MPa, or a ratio of the elastic modulus of the first elastic layer to the elastic modulus of the second elastic layer is greater than or equal to 10.

Because the elastic modulus of the first elastic layer is greater than the elastic modulus of the second elastic layer, the first elastic layer is made of a high modulus material, and the second elastic layer is made of a low modulus material. The low modulus material provides good stretchability, and ensures that the sensor has a good bending characteristic and a good stretching characteristic. In addition, the low modulus material can further provide good aqueous oxygen isolation, so that the ionic gel layer is not affected by aqueous oxygen. The high modulus material itself provides good resilience. Therefore, forming a composite structure by using the first elastic layer and the second elastic layer can improve performance stability of the sensor.

In a possible implementation, at least one of the first elastic layer and the second elastic layer is provided with a groove structure.

The at least one of the first elastic layer and the second elastic layer is provided with a groove structure, so that stretchability in a discontinuous direction can also be ensured to some extent, and a degree of irregular surface deformation of the packaging layer caused during bending is reduced, thereby further ensuring performance stability of the sensor.

A second aspect of this application provides a detection apparatus, including a pressurization member and the sensor according to any implementation of the first aspect. The pressurization member is configured to apply pressure to a measured site. The sensor is configured to measure a pulse wave signal at the measured site, and the sensor is disposed between the pressurization member and the measured site.

A third aspect of this application provides a wearable device, including a watch body, a first watch band, a second watch band, and the sensor according to any implementation of the first aspect. Two opposite ends of the watch body are respectively connected to one end of the first watch band and one end of the second watch band, and the sensor is connected to the first watch band.

A fourth aspect of this application provides an ionic gel, including an elastomer polymer skeleton and ionic liquid disposed in the elastomer polymer skeleton. The elastomer polymer skeleton includes 1,4-butanediol divinyl ether, 2,2'-(ethylenedioxy)diethanethiol, trimethylolpropane tris(3-mercaptopropionate), and benzoin dimethyl ether. The ionic liquid includes 1-ethyl-3-methylimidazolium bis(trifluoromethylsulfonyl)imide.

A fifth aspect of this application provides a preparation method for an ionic gel, including the following steps:
uniformly mixing 2,2'-(ethylenedioxy)diethanethiol, trimethylolpropane tris(3-mercaptopropionate), benzoin dimethyl ether, 1,4-butanediol divinyl ether, and 1-ethyl-3-methylimidazolium bis(trifluoromethylsulfonyl)imide to obtain a precursor solution; and
placing the precursor solution under ultraviolet light to perform polymerization reaction, to obtain the ionic gel.

In a possible implementation, uniformly mixing the 2,2'-(ethylenedioxy)diethanethiol, the trimethylolpropane tris(3-mercaptopropionate), the benzoin dimethyl ether, the 1,4-butanediol divinyl ether, and the 1-ethyl-3-methylimidazolium bis(trifluoromethylsulfonyl)imide to obtain the precursor solution includes the following steps:
mixing the 2,2'-(ethylenedioxy)diethanethiol, the trimethylolpropane tris(3-mercaptopropionate), the benzoin dimethyl ether, and the 1-ethyl-3-methylimidazolium bis(trifluoromethylsulfonyl)imide until the benzoin dimethyl ether is completely dissolved and a liquid mixture is obtained; and
adding the 1,4-butanediol divinyl ether to the liquid mixture to obtain the precursor solution.

In a possible implementation, a ratio of 2,2'-(ethylenedioxy)diethanethiol, trimethylolpropane tris(3-mercaptopropionate), benzoin dimethyl ether, and 1-ethyl-3-methylimidazolium bis(trifluoromethylsulfonyl)imide is 93:22:20:1.

In a possible implementation, a ratio of 1,4-butanediol divinyl ether to benzoin dimethyl ether is 102:1.

### BRIEF DESCRIPTION OF DRAWINGS

FIG. 1 is a diagram of a structure of a wearable device according to an embodiment of this application;
FIG. 2 is a diagram of a structure of a first sensor according to an embodiment of this application;
FIG. 3 is a cross-sectional diagram of the sensor in FIG. 2;
FIG. 4 is a diagram of a structure of a first support layer in FIG. 3;
FIG. 5 is a diagram of a structure of a second support layer according to an embodiment of this application;
FIG. 6 is a cross-sectional diagram of the sensor in FIG. 3 when the sensor is bent;
FIG. 7 is a cross-sectional diagram of a second sensor according to an embodiment of this application;
FIG. 8 is a cross-sectional diagram of a packaging layer from a top perspective according to an embodiment of this application;
FIG. 9 is a cross-sectional diagram of another packaging layer from a top perspective according to an embodiment of this application;
FIG. 10 is a cross-sectional diagram of a third sensor according to an embodiment of this application;
FIG. 11 is a cross-sectional diagram of an ionic gel layer being connected to a packaging layer by using a coupling agent layer in FIG. 10;
FIG. 12 is a cross-sectional diagram of a fourth sensor according to an embodiment of this application;
FIG. 13 is a cross-sectional diagram of a fifth sensor according to an embodiment of this application;
FIG. 14 is a cross-sectional diagram of a sixth sensor according to an embodiment of this application;
FIG. 15 is a cross-sectional diagram of a seventh sensor according to an embodiment of this application; and
FIG. 16 is a cross-sectional diagram of an eighth sensor according to an embodiment of this application.

### Description of reference numerals:

100: sensor;
10: substrate layer;
20: electrode layer;
30: ionic gel layer; 31: microstructure;
40: packaging layer; 41: first elastic layer; 411: strip portion; 412: columnar portion; 42: second elastic layer; 43: groove structure; 431: strip groove;
50: support layer; 51: through hole;
60: coupling agent layer;
70: bonding layer;
200: pressurization member; 210: first watch band; 220: second watch band; 230: airbag;
300: detection apparatus;
400: watch body; and
500: wearable device.

### DESCRIPTION OF EMBODIMENTS

In a related technology, a wearable device includes a pressurization mechanism and a piezoelectric pressure sensor. During a process in which the pressurization mechanism applies pressure to a wrist of a subject, a piezoelectric pressure sensor can detect changes in a pulse signal at the wrist. However, because a piezoelectric pressure sensor cannot detect a static force, to implement a blood pressure detection function, a pressure sensor for detecting a static force needs to be added. Therefore, to implement the blood pressure detection function, two pressure sensors need to be used.

In view of this, embodiments of this application provide a sensor 100, a detection apparatus 300, a wearable device 500, an ionic gel, and a preparation method. The sensor 100 outputs a capacitance value by using an ion-electron pair, so that a dynamic force and a static force can be detected. In addition, because a spacing between ion-electron pairs is far less than a thickness of a dielectric layer in a conventional parallel-plate capacitive force sensor, the capacitance value output by the sensor 100 is several orders of magnitude higher than that of a parallel-plate capacitor, increasing from a pF level to an nF level. This also improves sensitivity and force resolution of the sensor 100 by 10 to 100 times.

The sensor 100 provided in embodiments of this application may be used for the wearable device 500 or the detection apparatus 300. This is not specifically limited herein.

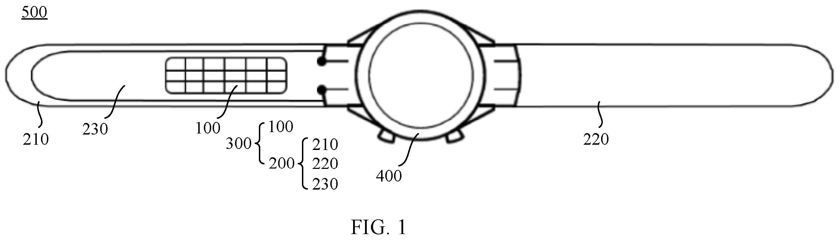

The wearable device 500 may include but is not limited to a smart band, a smartwatch, a watch, a watch-type blood pressure meter, or the like. In embodiments of this application, description is provided by using an example in which the wearable device 500 is a smartwatch, as shown in FIG. 1.

FIG. 1 is a diagram of a structure of a wearable device according to an embodiment of this application.

Referring to FIG. 1, the wearable device 500 in embodiments of this application includes a watch body 400, a pressurization member 200, and a sensor 100. The pressurization member 200 includes a first watch band 210, a second watch band 220, and an airbag 230. Two opposite ends of the watch body 400 are respectively connected to one end of the first watch band 210 and one end of the second watch band 220, the other end of the first watch band 210 is detachably connected to the other end of the second watch band 220, the sensor 100 is connected to the first watch band 210, and the airbag 230 is disposed between the first watch band 210 and the sensor 100.

The airbag 230 is configured to apply pressure to a measured site of a subject. The sensor 100 is located between the measured site and the airbag 230 and measures a pulse wave signal of the measured site. The wearable device 500 obtains blood pressure and a heart rate of the subject based on the pulse wave signal. The airbag 230 applies a plurality of different pressure values to the measured site, so that an envelope line of the pulse wave signal changes, and a blood pressure value can be calculated based on the change in the envelope line. The airbag 230 applies fixed pressure to the measured site, to collect statistics about frequencies of the pulse wave signal, and a heart rate value can be calculated based on the frequencies of the pulse wave signal.

An embodiment of this application further provides a detection apparatus 300. The detection apparatus 300 includes a pressurization member 200 and a sensor 100. The pressurization member 200 is configured to apply pressure to the measured site. The sensor 100 is configured to measure a pulse wave signal of a measured site, and the sensor 100 is disposed between the pressurization member 200 and the measured site.

The detection apparatus 300 may be a blood pressure meter, a pressure detection device, or the like. This is not specifically limited herein.

A specific structure of the pressurization member 200 is not limited herein. In some implementations, the pressurization member 200 is a stretchable watch band. In this case, the pressurization member 200 may form an annular structure surrounding a measured site of a subject. When pressure is applied to the measured site of the subject, an inner diameter of the pressurization member 200 becomes larger or smaller, so that the force applied to the measured site becomes larger or smaller.

In some other implementations, the pressurization member 200 may further include an airbag 230, and the airbag 230 is configured to form an annular structure surrounding the measured site of the subject. Pressure is applied to the measured site by inflating and deflating the airbag 230.

In some other implementations, the pressurization member 200 may further include a first watch band 210, a second watch band 220, and an airbag 230. One end of the first watch band 210 is connected to one end of the second watch band 220, the other end of the first watch band 210 is detachably connected to the other end of the second watch band 220, and the airbag 230 is connected to the first watch band 210.

With reference to the accompanying drawings, the following describes an implementation of the sensor 100 provided in embodiments of this application.

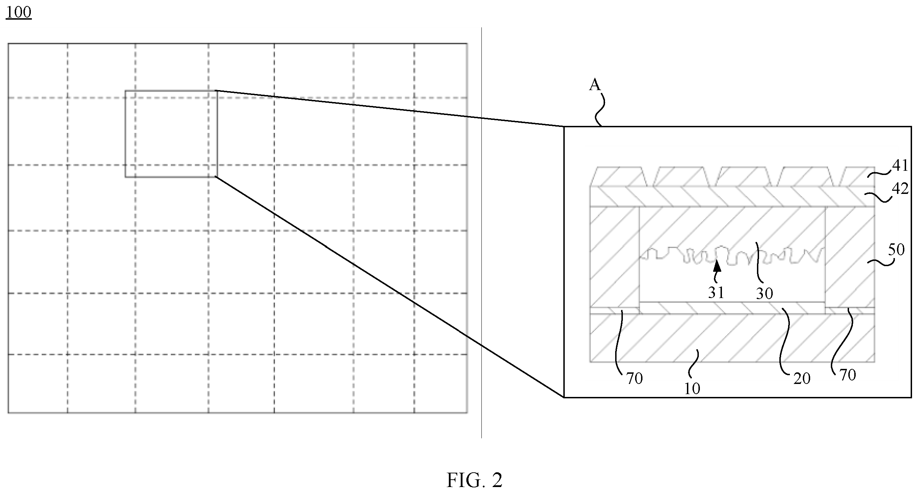

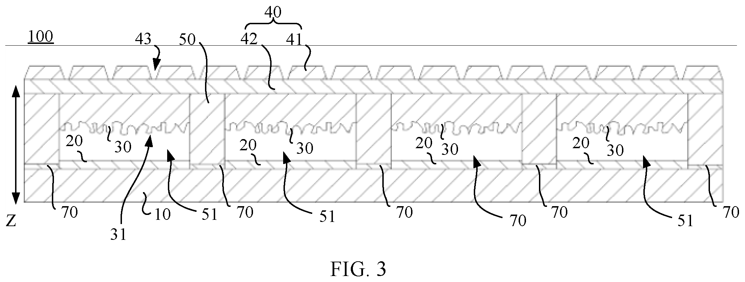

FIG. 2 is a diagram of a structure of a first sensor according to an embodiment of this application. FIG. 3 is a cross-sectional diagram of the sensor in FIG. 2.

Referring to FIG. 3, the sensor 100 in embodiments of this application includes a support layer 50, a substrate layer 10, an electrode layer 20, an ionic gel layer 30, and a packaging layer 40. The electrode layer 20 is disposed between the ionic gel layer 30 and the substrate layer 10, the ionic gel layer 30 is disposed between the electrode layer 20 and the packaging layer 40, and the packaging layer 40 is connected to the substrate layer 10 through the support layer 50. The sensor 100 is configured to receive a pressing force, so that a contact area between the ionic gel layer 30 and the electrode layer 20 changes, and an ion-electron pair formed at a contact interface between the ionic gel layer 30 and the electrode layer 20 changes.

During a process in which the sensor 100 detects the pulse wave signal, the packaging layer 40 is in contact with the measured site of the subject, the substrate layer 10 is connected to the pressurization member 200, and a contact area between the ionic gel layer 30 and the electrode layer 20 changes during a process in which the pressurization member 200 applies pressure to the measured site of the subject, so that a quantity of ion-electron pairs formed at the contact interface between the ionic gel layer 30 and the electrode layer 20 changes, a capacitance value output by the sensor 100 changes, and static and dynamic payload values can be simultaneously detected.

When the pressing force is a static force, the ionic gel layer 30 can come into contact with the electrode layer 20, and an ion-electron pair is formed at a contact interface between the ionic gel layer 30 and the electrode layer 20, so that the sensor 100 outputs a capacitance value, thereby implementing force detection. When the pressing force is a dynamic force, the contact area between the ionic gel layer 30 and the electrode layer 20 changes with the pressing, so that a quantity of ion-electron pairs formed at the contact interface between the ionic gel layer 30 and the electrode layer 20 changes, and as a result, the capacitance value output by the sensor 100 changes, thereby implementing force detection. Therefore, the sensor 100 provided in embodiments of this application can detect static and dynamic forces, and a pressure sensor for detecting a static force may be removed. This can help reduce costs of the wearable device 500, or help implement miniaturization of the wearable device 500.

In addition, compared with a conventional parallel-plate capacitive force sensor, a change in the capacitance value output by the sensor 100 provided in embodiments of this application depends on a change in the quantity of ion-electron pairs at a contact interface between an ionic gel and an electrode. Because a spacing between ion-electron pairs is far less than a thickness of a dielectric layer in a conventional parallel-plate capacitor, the capacitance value output by the sensor 100 provided in embodiments of this application is several orders of magnitude higher than that of the parallel-plate capacitor, and is increased from a pF level to an nF level. This also improves sensitivity and force resolution of the sensor 100 by 10 to 100 times.

In embodiments of this application, the substrate layer 10 is a base of the sensor 100, and provides support for all sensing structures. In addition, the substrate layer 10 further provides a function of packaging the sensor 100. In addition, a material of a substrate may include but is not limited to a polymer material such as polyethylene naphthalate (polyethylene naphthalate, PEN), polyethylene glycol terephthalate (polyethylene glycol terephthalate, PET), polyimide (polyimide, PI), polystyrene (polystyrene, PS), polyvinyl chloride (polyvinyl chloride, PVC), or polydimethylsiloxane (polydimethylsiloxane, PDMS).

In embodiments of this application, a material of the electrode layer 20 may include but is not limited to a conductor material such as gold (Au), silver (Ag), copper (Cu), aluminum (Al), graphite (C), or silver nanowire.

In some implementations, the electrode layer 20 may be integrated on a surface of the substrate layer 10 in a manner of surface attachment, deposition, or the like.

A specific shape of the electrode layer 20 is not limited herein. In some implementations, a morphology of the electrode layer 20 may be an entire sheet-like electrode. In some other implementations, the electrode layer 20 may alternatively be a finger-shaped or comb-shaped interdigital electrode.

In embodiments of this application, a material of the support layer 50 may include but is not limited to a polymer material such as polyethylene naphthalate (PEN), polyethylene glycol terephthalate (PET), polyimide (PI), polystyrene (PS), polyvinyl chloride (PVC), or polydimethylsiloxane (PDMS).

In some possible implementations, as shown in FIG. 3, a microstructure 31 is disposed on a surface of the ionic gel layer 30 facing the electrode layer 20, and the microstructure 31 ensures that the surface of the ionic gel layer 30 facing the electrode layer 20 is rough (concave-convex) or uneven. In other words, the surface of the ionic gel layer 30 facing the electrode layer 20 is a non-smooth surface. Therefore, when the sensor 100 is pressed, the contact area between the ionic gel layer 30 and the electrode layer 20 may be expanded, and the quantity of ion-electron pairs may be increased, so that the capacitance value output by the sensor 100 is increased, thereby helping improve sensitivity and force resolution of the sensor 100.

A specific structure of the microstructure 31 is not limited herein. In some implementations, as shown in FIG. 3, the microstructure 31 may be a sandpaper structure. In some other implementations, the microstructure 31 may alternatively include a plurality of bump structures disposed on the surface of the ionic gel layer 30 facing the electrode layer 20, and the bump structures may be in a conical shape.

How to form the microstructure 31 on the ionic gel layer 30 is not limited herein. In some implementations, a preparation manner of the microstructure 31 on the surface of the ionic gel layer 30 may be as follows: A precursor solution of the ionic gel layer 30 (referring to the following description about a preparation process of an ionic gel) is poured into a mold with a sandpaper structure. A support layer 50 of a grid structure has been pre-mounted in the mold. Therefore, the precursor solution of the ionic gel layer 30 is distributed in an independent "microwell (which may be understood as a through hole 51 of the support layer 50)". Then, the packaging layer 40 with a bonding modification material is pressed on the precursor solution of the ionic gel layer 30, the precursor solution of the ionic gel layer 30 is irradiated by using an ultraviolet lamp to cure the precursor solution, and the packaging layer 40 is removed from the mold, to obtain the ionic gel layer 30 that is bonded to the packaging layer 40 and that has a sandpaper microstructure 31 on a surface.

It should be noted that, in addition to disposing the microstructure 31 on the ionic gel layer 30, in some implementations, the microstructure 31 (not shown in the figure) may also be disposed on a surface of the electrode layer 20 facing the ionic gel layer 30. In this way, the contact area between the electrode layer 20 and the ionic gel layer 30 may also be expanded. In addition, the microstructure 31 may be disposed in at least one of the ionic gel layer 30 and the electrode layer 20. For example, the microstructure 31 is disposed in both the ionic gel layer 30 and the electrode layer 20, or the microstructure 31 is disposed in one of the ionic gel layer 30 and the electrode layer 20.

In some possible implementations, as shown in FIG. 3, the ionic gel layer 30 and the electrode layer 20 are disposed at intervals in a first direction Z, and the first direction Z is a thickness direction of the substrate layer 10. In this way, when the sensor 100 is pressed, the contact area between the ionic gel layer 30 and the electrode layer 20 may be expanded, and the quantity of ion-electron pairs may be increased, so that the capacitance value output by the sensor 100 is increased, thereby helping improve sensitivity and force resolution of the sensor 100.

The ionic gel layer 30 is connected to the packaging layer 40, the electrode layer 20 is connected to the substrate layer 10, and the packaging layer 40 is connected to the substrate layer 10 through the support layer 50. Therefore, in addition to connecting the substrate layer 10 and the packaging layer 40, the support layer 50 is further configured to separate the ionic gel layer 30 from the electrode layer 20. The electrode layer 20 is in contact with the ionic gel layer 30 only when the electrode layer is pressed. In addition, a larger pressing force indicates a larger contact area between the electrode layer 20 and the ionic gel layer 30, and a larger capacitance value output by the sensor 100. Therefore, presence of the support layer 50 can ensure normal sensing performance of the sensor 100.

In some possible implementations, as shown in FIG. 3, the support layer 50 is provided with a through hole 51, the through hole 51 runs through the support layer 50 in the first direction Z, an axial direction of the through hole 51 is parallel to the first direction Z, and the ionic gel layer 30 and the electrode layer 20 are disposed inside the through hole 51. In this way, the support layer 50 can protect the electrode layer 20 and the ionic gel layer 30, and the ionic gel layer 30 can come into contact with the electrode layer 20 when the sensor 100 is pressed, to form an ion-electron pair.

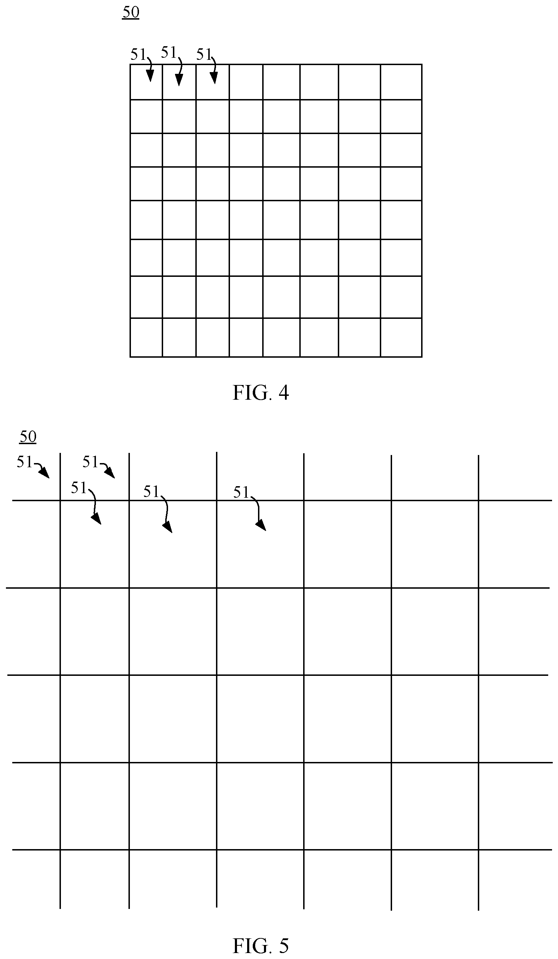

A specific shape of the through hole 51 is not limited herein. In some implementations, the through hole 51 may be a circular through hole 51 (not shown in the figure). In some other implementations, the through hole 51 may alternatively be a square through hole (as shown in FIG. 4).

FIG. 4 is a diagram of a structure of a first support layer in FIG. 3.

As shown in FIG. 4, the support layer 50 includes a plurality of through holes 51 disposed at intervals, so that the support layer 50 is of a grid structure. As shown in FIG. 3, one ionic gel layer 30 and one electrode layer 20 are disposed in each through hole 51. Therefore, there are a plurality of ionic gel layers 30 and a plurality of electrode layers 20, adjacent ionic gel layers 30 are separated through the support layer 50, and adjacent electrode layers 20 are separated through the support layer 50.

The ionic gel layer 30 and the electrode layer 20 in each through hole 51 may form one matrix point, so that the sensor 100 is equivalent to being formed by combining a plurality of arrays of matrix points. The grid structure of the support layer 50 can ensure that various matrix points form a "microwell" structure (as shown in A in FIG. 2), so that ionic gel materials in the structure are separated from each other, thereby ensuring normal sensing performance of the sensor 100 and effectively eliminating mutual crosstalk between matrix points.

A function of the support layer 50 is that the support layer 50 separates the ionic gel layer 30 from the electrode layer 20, and the ionic gel layer 30 comes into contact with the electrode layer 20 only when the ionic gel layer is pressed by an external force, and generates a capacitance change that varies with pressure, thereby ensuring normal performance of the sensor 100. Another function is that the support layer 50 in a grid shape separates matrix points in an array from each other, and an ionic gel layer 30-electrode layer 20 combination in each matrix point can independently output one group of capacitance values, and does not affect capacitance values of an ionic gel layer 30-electrode layer 20 combination in an adjacent matrix point. Therefore, there is no crosstalk between matrix points, thereby ensuring measurement accuracy.

Because adjacent electrode layers 20 are separated by the support layer 50, there is a specific gap between adjacent electrode layers 20, and a trace (not shown in the figure) of the electrode layer 20 may be routed in the gap. In addition, the support layer 50 may alternatively be connected to the substrate layer 10 by using the gap.

A specific structure of the support layer 50 is not limited herein. In some implementations, as shown in FIG. 4, each of the plurality of through holes 51 is formed by using four side walls. In some other implementations, as shown in FIG. 5, some of the plurality of through holes 51 are formed by using four side walls, and some other through holes 51 are formed by using two side walls or three side walls. FIG. 5 is a diagram of a structure of a second support layer according to an embodiment of this application.

In some implementations, as shown in FIG. 3, the sensor 100 may further include a bonding layer 70. The bonding layer 70 is disposed between the support layer 50 and the substrate layer 10, so that the support layer 50 is connected to the substrate layer 10 in a bonding manner.

As shown in FIG. 4, because the support layer 50 is of a grid structure, a structure of the bonding layer 70 is also of a grid structure, and a grid size of the bonding layer 70 matches a grid size of the support layer 50, to ensure that the support layer 50 is connected to the bonding layer 70.

A specific material of the bonding layer 70 is not limited herein. For example, the bonding layer 70 may be a double-sided tape. When the bonding layer 70 is a double-sided tape, the double-sided tape may be attached to the support layer 50 in advance, and then the double-sided tape and the support layer 50 of the grid structure are machined.

For example, ion cleaning may be first performed on the support layer 50 and the double-sided tape in a sheet shape, then the support layer 50 and the double-sided tape are pressed together, and then the support layer 50 and the double-sided tape that are connected are placed in an oven for heating for a specific time based on a specific temperature until the support layer 50 and the double-sided tape of the grid structure are cut by using a laser after cooling.

A grid size of the support layer 50 and a grid size of the double-sided tape are kept consistent with a gap size of the electrode layer 20, to ensure that the support layer 50 of the grid structure is distributed in the gap between electrode layers 20, and that the double-sided tape of the grid structure is bonded to the substrate layer 10 by using the gap between electrode layers 20, so as to connect the support layer 50 to the substrate layer 10.

In embodiments of this application, a specific material of the ionic gel layer 30 is not specifically limited herein. The ionic gel layer 30 may be obtained by mixing a plurality of organic materials.

In some possible implementations, the ionic gel layer 30 may include 1,4-butanediol divinyl ether, 2,2'-(ethylenedioxy)diethanethiol, trimethylolpropane tris(3-mercaptopropionate), benzoin dimethyl ether, and 1-ethyl-3-methylimidazolium bis(trifluoromethylsulfonyl)imide.

Specifically, the ionic gel layer 30 is formed by an elastomer polymer skeleton that is generated through click polymerization reaction by using 1,4-butanediol divinyl ether (1,4-butanediol divinyl ether, BVB), 2,2'-(ethylenedioxy)diethanethiol (2,2'-(ethylenedioxy)diethanethiol, BMOE), and trimethylolpropane tris(3-mercaptopropionate) (trimethylolpropane tris(3-mercaptopropionate), TMPT) as monomers, and by using benzoin dimethyl ether (2,2-dimethoxy-2-phenylacetophenone, DMPA) as a photoinitiator, and ionic liquid such as 1-ethyl-3-methylimidazolium bis(trifluoromethylsulfonyl)imide (1-ethyl-3-methylimidazolium bis(trifluoromethylsulfonyl)imide, EMIM[TFSI]) that is uniformly distributed in the polymer skeleton. In the sensor 100, the ionic gel layer 30 made of these materials is in contact with the electrode layer 20, so that a quantity of ion-electron pairs formed when the ionic gel layer 30 is in contact with the electrode layer 20 can be increased, and sensitivity and force resolution of the sensor 100 can be further improved.

It should be noted that, in addition to using the ionic gel layer 30 that is made of these materials, the sensor 100 may alternatively use an ionic gel layer 30 that is made of another material.

In embodiments of this application, the packaging layer 40 is configured to provide functions of anti-wear, waterproofing, and oxygen isolation for the sensor 100. A specific structure of the packaging layer 40 is not limited herein.

In some possible implementations, as shown in FIG. 3, the packaging layer 40 includes a first elastic layer 41 and a second elastic layer 42. The second elastic layer 42 is disposed between the first elastic layer 41 and the ionic gel layer 30 and is bonded to the ionic gel layer 30. An elastic modulus of the first elastic layer 41 is greater than or equal to 1 GPa and an elastic modulus of the second elastic layer 42 is less than or equal to 100 MPa, or a ratio of the elastic modulus of the first elastic layer 41 to the elastic modulus of the second elastic layer 42 is greater than or equal to 10.

It should be noted that a relationship between the elastic modulus of the first elastic layer 41 and the elastic modulus of the second elastic layer 42 meets at least one of the following two conditions: 1. The elastic modulus of the first elastic layer 41 is greater than or equal to 1 GPa, and the elastic modulus of the second elastic layer 42 is less than or equal to 100 MPa. 2. The ratio of the elastic modulus of the first elastic layer 41 to the elastic modulus of the second elastic layer 42 is greater than or equal to 10.

The first elastic layer 41 is made of a high modulus material. The high modulus material is a polymer material whose elastic modulus is greater than or equal to 1 GPa, and includes but is not limited to polyethylene glycol terephthalate (PET), polyethylene naphthalate (PEN), and the like. The material with a high elastic modulus can quickly rebound when bending or pressing pressure is removed, that is, the material of this type has good creep resistance. This helps implement a basic function of the sensor 100: The packaging layer 40 and the ionic gel layer 30 connected to the packaging layer generate corresponding bending deformation under an applied pressure, so as to ensure that there is a corresponding contact area between the ionic gel layer 30 and the electrode layer 20, and a corresponding capacitance value is output. When the applied pressure changes quickly, for example, the pressure decreases quickly, the material with a high elastic modulus in the packaging layer 40 quickly rebounds, the corresponding bending deformation decreases, the contact area between the ionic gel layer 30 and the electrode layer 20 decreases, and the capacitance value output by the sensor 100 decreases, thereby enabling signal sensing of a decrease in the force.

The second elastic layer 42 is made of a low modulus material. The low modulus material is a polymer material whose elastic modulus is less than or equal to 100 MPa, and includes but is not limited to polydimethylsiloxane (PDMS), Ecoflex, a silica gel, and the like. The material with a low elastic modulus has good stretchability, and can ensure that the sensor 100 has a good bending characteristic and a good stretching characteristic. In addition, the material with a low elastic modulus can further provide good aqueous oxygen isolation, thereby ensuring stability of the ionic gel layer 30. In addition, in a preparation process of the sensor 100, the material with a low elastic modulus itself is cleaned by using Plasma plasma, and an oxygen chain side chain (-O-) on a material on a side of the material with a low elastic modulus near the ionic gel layer 30 is changed to a hydroxyl side chain (-OH), so that the material with a low elastic modulus can be combined with a coupling agent layer 60 (refer to the following description), thereby ensuring that the material with a low elastic modulus can be firmly attached to the ionic gel layer 30.

In conclusion, the packaging layer 40 is made of a material with a low elastic modulus and a material with a high elastic modulus. The low modulus material provides good stretchability, and ensures that the sensor 100 has a good bending characteristic and a good stretching characteristic. In addition, the low modulus material can further provide good aqueous oxygen isolation, so that the ionic gel layer 30 is not affected by aqueous oxygen. The high modulus material itself provides good resilience, and can improve performance stability of the sensor 100. Therefore, the packaging layer 40 formed by the first elastic layer 41 and the second elastic layer 42 can ensure that the sensor 100 can quickly respond, and further ensure repeated and stable sensing performance of the sensor 100.

In some possible implementations, at least one of the first elastic layer 41 and the second elastic layer 42 is provided with a groove structure 43. For example, as shown in FIG. 3, the first elastic layer 41 is provided with a groove structure 43, and the second elastic layer 42 is not provided with a groove structure 43.

Because the first elastic layer 41 is formed by a material with a high elastic modulus and the second elastic layer 42 is formed by a material with a low elastic modulus, when the two materials with different elastic moduli are bent under force, different bending states (with different curvatures, different curvature distributions, and the like) are easily generated, and irregular surface deformation is easily caused, affecting performance of the sensor 100. Therefore, the first elastic layer 41 is provided with the groove structure 43 to pattern the first elastic layer 41, so that irregular surface deformation can be reduced or eliminated, thereby ensuring resilience and stretchability of the packaging layer 40.

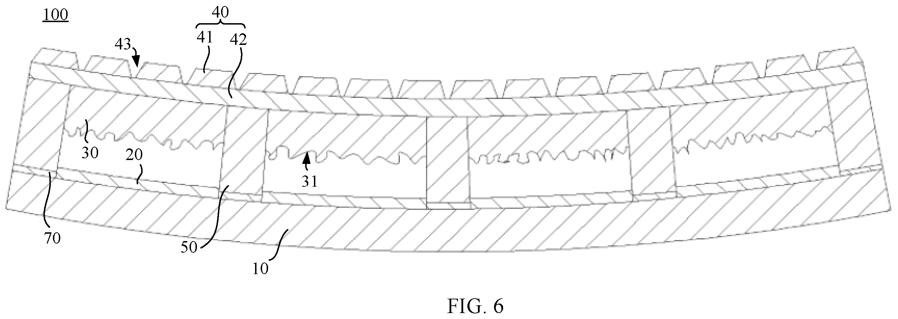

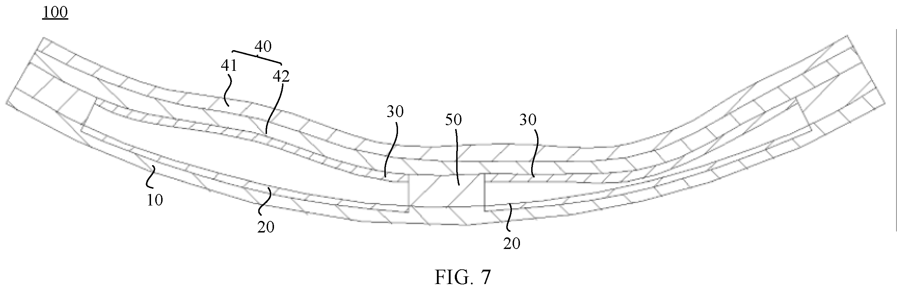

FIG. 6 is a cross-sectional diagram of the sensor in FIG. 3 when the sensor is bent. FIG. 7 is a cross-sectional diagram of a second sensor according to an embodiment of this application. FIG. 7 differs from FIG. 6 in that neither the first elastic layer 41 nor the second elastic layer 42 in FIG. 7 is provided with a groove structure 43.

In some implementations, a part of the first elastic layer 41 may be cut or etched to form a strip-shaped array structure or a stretchable grid structure, to form a patterned structure. An effect of this structure is that because the material of the first elastic layer 41 is a material with a high elastic modulus, the first elastic layer has a good rebound characteristic in a continuous direction of the material. In a discontinuous direction (a direction in which cutting or etching is performed) of the material, the material has stretchability. In addition, because a part of the material is removed, when the material undergoes bending deformation, an internal stress of the material is less than that of the entire material that is not patterned. Therefore, an amount of bending deformation of the material is less than an amount of deformation of the entire material. When the material is combined with a material with a low elastic modulus, a degree of caused irregular surface deformation is reduced (as shown in FIG. 6). This may be significantly different from a case of large surface deformation shown in FIG. 7. Therefore, the patterned structure can ensure resilience of the packaging layer 40, and can further ensure that the packaging layer has stretchability, and reduce irregular surface deformation between two layers during bending deformation.

In this example of this application, a specific structure of the first elastic layer 41 having the groove structure 43 is not specifically limited herein.

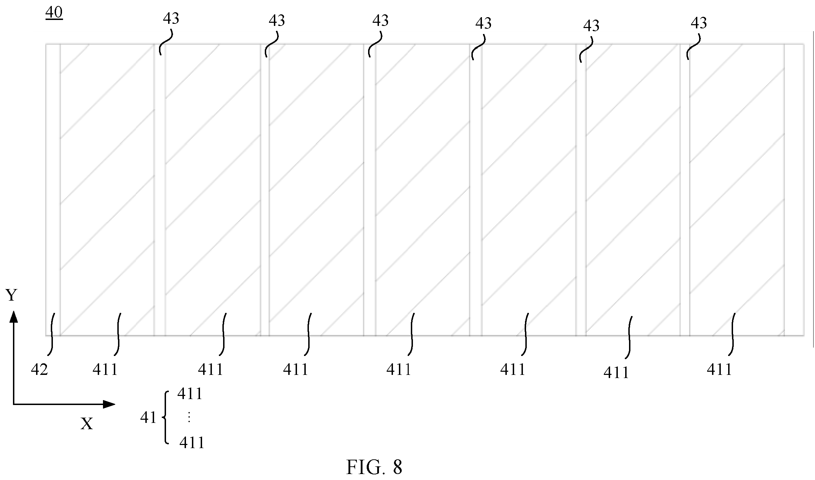

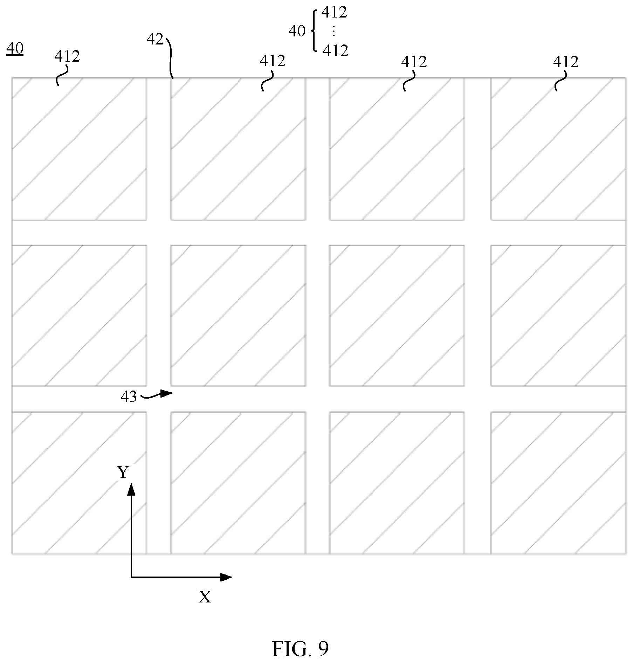

FIG. 8 is a cross-sectional diagram of a packaging layer from a top perspective according to an embodiment of this application. FIG. 9 is a cross-sectional diagram of another packaging layer from a top perspective according to an embodiment of this application.

In some implementations, as shown in FIG. 8, the first elastic layer 41 may include a plurality of strip portions 411 that are arranged side by side and at intervals in a second direction X, an extension direction of each strip portion 411 is parallel to a third direction Y, and one groove structure 43 is formed between two adjacent strip portions 411.

In some other implementations, as shown in FIG. 9, the first elastic layer 41 may include a plurality of columnar portions 412 disposed at intervals, the plurality of columnar portions 412 are arranged in an array in the second direction X and the third direction Y, and a gap between adjacent columnar portions 412 is used to form a groove structure 43.

In some other implementations, the first elastic layer 41 may alternatively include a body portion and a plurality of strip portions 411. The body portion is connected to the second elastic layer 42, the plurality of strip portions 411 are disposed on a same side of the body portion, the plurality of strip portions 411 are arranged side by side and at intervals in the second direction X, each strip portion 411 extends in the third direction Y, and two adjacent strip portions 411 and the body portion form one groove structure 43.

In this example of this application, the first direction Z, the second direction X, and the third direction Y are perpendicular to each other. The first direction Z is a thickness direction of the substrate layer 10.

It should be noted that, in addition to the first elastic layer 41 being provided with the groove structure 43, in some implementations, alternatively, the second elastic layer 42 may be provided with a groove structure 43, and the first elastic layer 41 is not provided with a groove structure 43. In some other implementations, alternatively, both the first elastic layer 41 and the second elastic layer 42 may be provided with a groove structure 43.

When the second elastic layer 42 is provided with the groove structure 43, in the first direction Z, the groove structure 43 does not run through the second elastic layer 42. In other words, the groove structure 43 is not a through groove structure. Alternatively, it may be understood that when the second elastic layer 42 is provided with the groove structure 43, the second elastic layer 42 is still an integrated plate structure, but is not formed by a plurality of discrete portions. This can ensure that the second elastic layer 42 can be connected to the ionic gel layer 30.

When the second elastic layer 42 is provided with the groove structure 43, the groove structure 43 is disposed on a side of the second elastic layer 42 facing the first elastic layer 41 in the first direction Z. In this case, a part of the first elastic layer 41 may be disposed inside the groove structure 43, so as to expand a contact area between the first elastic layer 41 and the second elastic layer 42, thereby helping improve firmness of connection between the first elastic layer 41 and the second elastic layer 42.

In embodiments of this application, the ionic gel layer 30 may be connected to the packaging layer 40 in a bonding manner. In some implementations, the ionic gel layer 30 may be bonded to the packaging layer 40 by using a bonding member (not shown in the figure) such as a double-sided tape, glue, or an adhesive tape. In some other implementations, the ionic gel layer 30 may alternatively be connected to the packaging layer 40 through chemical bonding, so that the ionic gel layer 30 is chemically bonded to the packaging layer 40, and the ionic gel layer 30 and the packaging layer 40 form an integrated structure.

Correspondingly, the ionic gel layer 30 is connected to the packaging layer 40 by using a bonding member or in a chemical bonding manner, which can ensure that there is a sufficient bonding force between the ionic gel layer 30 and the packaging layer 40, so that the ionic gel layer 30 and the packaging layer 40 are bonded together, and do not detach during stretching or bending, thereby ensuring stability of the sensor 100 during long-term use.

Because a thickness (usually at a hundred-micron level) of the bonding member in the first direction Z is relatively large, a thickness and bendability of the sensor 100 in the first direction Z are affected. However, when the ionic gel layer 30 is connected to the packaging layer 40 in a chemical bonding manner, a thickness of a chemical layer between the ionic gel layer 30 and the packaging layer 40 may be controlled to be a thickness at a submicron (less than µm) level, and a thickness, stretching, and bending performance of the sensor 100 are not affected, so that a thickness of the sensor 100 in the first direction Z may be made smaller. Therefore, in this example of this application, the ionic gel layer 30 and the packaging layer 40 form an integrated structure in a chemical bonding manner.

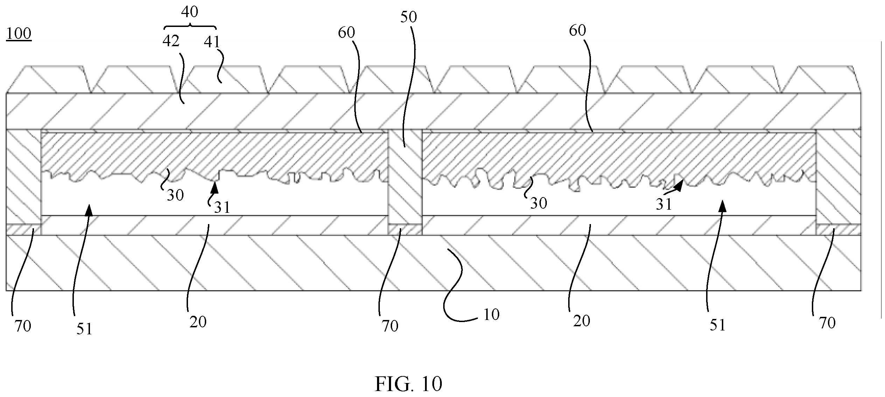

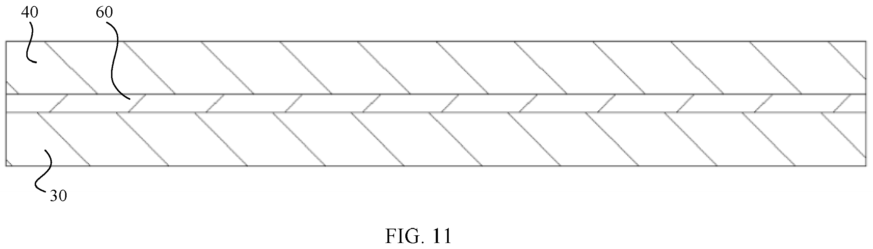

FIG. 10 is a cross-sectional diagram of a third sensor according to an embodiment of this application. FIG. 11 is a cross-sectional diagram of an ionic gel layer being connected to a packaging layer by using a coupling agent layer in FIG. 10.

In some possible implementations, as shown in FIG. 10, the sensor 100 may further include a coupling agent layer 60, the coupling agent layer 60 is disposed between the ionic gel layer 30 and the packaging layer 40, the ionic gel layer 30 is connected to the coupling agent layer 60 through chemical bonding, and the packaging layer 40 is connected to the coupling agent layer 60 through chemical bonding. In this way, the ionic gel layer 30 may be chemically bonded to the packaging layer 40 by using the coupling agent layer 60, thereby ensuring that there is a sufficient bonding force between the packaging layer 40 and the ionic gel layer 30, and reducing a thickness of the sensor 100 in the first direction Z, without affecting bendability of the sensor 100.

A specific type of the coupling agent layer 60 is not limited herein. For example, the ionic gel layer 30 may form an integrated structure with the packaging layer 40 by using a coupling agent with mercapto, for example, (3-mercaptopropyl) trimethoxysilane, and perform chemical bonding with the packaging layer 40 and the ionic gel layer 30 separately through dehydration/dealcoholization reaction and click reaction, to separately implement a strong connection between the packaging layer 40-coupling agent bonding layer 70 (as shown in FIG. 11) and the gel layer-coupling agent bonding layer 70 (as shown in FIG. 11). Therefore, the ionic gel layer 30 and the packaging layer 40 are bonded together, and do not detach during stretching or bending, thereby ensuring stability of the sensor 100 during long-term use.

The following describes, by using an example in which the coupling agent layer 60 is (3-mercaptopropyl) trimethoxysilane, how the coupling agent layer 60 bonds the ionic gel layer 30 and the packaging layer 40 to form an integrated structure.

As shown in FIG. 10, the packaging layer 40 is formed by a first elastic layer 41 and a second elastic layer 42. The second elastic layer 42 is connected to the ionic gel layer 30. A material of the second elastic layer 42 may include but is not limited to a polymer with an oxygen chain (-O-), such as PDMS, Ecoflex, and a silica gel.

In a preparation process of the sensor 100, the packaging layer 40 is first cleaned by using Plasma plasma, so that an oxygen chain side chain (-O-) on the second elastic layer 42 is changed to a hydroxyl side chain (-OH), and then the packaging layer 40 is placed in a treatment solution (main components: (3-mercaptopropyl) trimethoxysilane + acetic acid) of a coupling agent for 30 minutes. Dealcoholization reaction occurring during this period is:

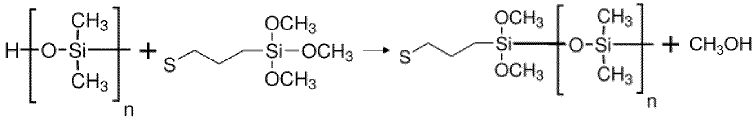

After the foregoing treatment steps, chemical bonding between the packaging layer 40 and the coupling agent layer 60 is completed, and a strong chemical bond is formed between the two layers to connect the two layers. Then, the coupling agent is chemically bonded to a monomer in the precursor solution of the ionic gel layer 30 by using a click chemistry (Click) reaction principle, so that the ionic gel layer 30 can be closely connected to the packaging layer 40 during curing.

In a preparation process of the sensor 100, the foregoing surface of the packaging layer 40 with the coupling agent layer 60 is pressed on the precursor solution of the ionic gel layer 30, and then irradiated by using an ultraviolet lamp, so that the precursor solution of the ionic gel layer 30 is cured. The click chemistry reaction occurring during this period is:

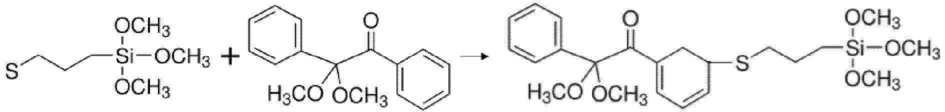

It can be seen from the reaction formula that, click reaction occurs on the mercapto (-SH) in the coupling agent layer 60 and a carbon-carbon double bond (C=C) in the monomer in the precuring solution of the ionic gel layer 30, to generate a stable carbon-sulfur bond (C-S), so that the coupling agent layer 60 is closely connected to the ionic gel layer 30.

In conclusion, due to presence of the coupling agent layer 60, a stable bonding structure (as shown in FIG. 11) of the packaging layer 40-the coupling agent layer 60-the ionic gel layer 30 is formed at a connection interface between the packaging layer 40 and the ionic gel layer 30, so that the ionic gel layer 30 can be firmly and stably bonded to the packaging layer 40.

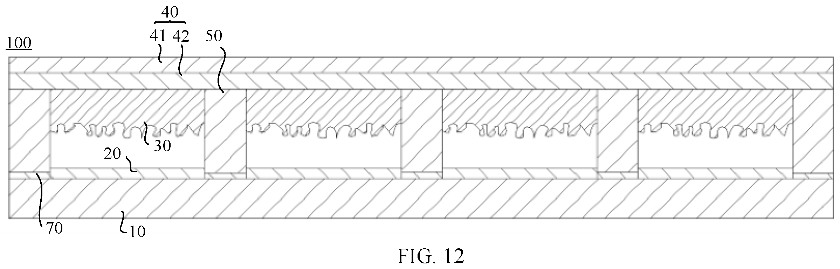

FIG. 12 is a cross-sectional diagram of a fourth sensor according to an embodiment of this application.

FIG. 12 differs from FIG. 3 in that the packaging layer 40 is also formed by the first elastic layer 41 and the second elastic layer 42, and neither the first elastic layer 41 nor the second elastic layer 42 is provided with a groove structure 43. In such an arrangement, the packaging layer 40 formed by the material with a high elastic modulus and the material with a low elastic modulus may also enable the sensor 100 to have good stretchability, bending performance, and resilience, thereby ensuring performance stability of the sensor 100.

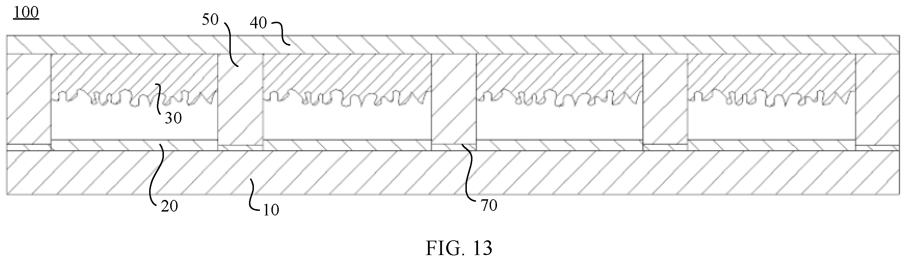

In the foregoing content, the packaging layer 40 is of a two-layer structure, and is formed by two materials with different elastic moduli. However, the packaging layer 40 may alternatively be of a single-layer structure, and the packaging layer 40 is formed by one material with an elastic modulus, as shown in FIG. 13. FIG. 13 is a cross-sectional diagram of a fifth sensor according to an embodiment of this application.

In FIG. 13, the packaging layer 40 may be bonded to the ionic gel layer 30 by using a bonding member or bonded to the ionic gel layer 30 in a chemical bonding manner.

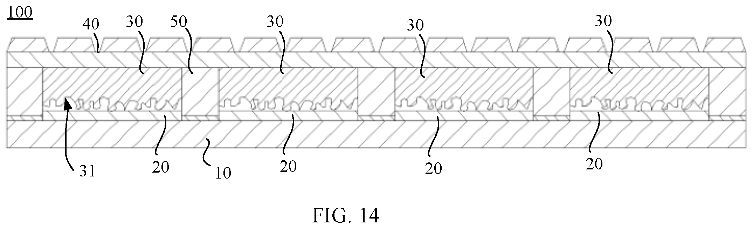

FIG. 14 is a cross-sectional diagram of a sixth sensor according to an embodiment of this application.

FIG. 14 differs from FIG. 3 in that the ionic gel layer 30 is in contact with the electrode layer 20 in the first direction Z. Specifically, before the sensor 100 is pressed, the ionic gel layer 30 keeps in contact with the electrode layer 20. Because a microstructure 31 is disposed on a surface of the ionic gel layer 30 facing the electrode layer 20, when the sensor 100 is pressed, a contact area between the ionic gel layer 30 and the electrode layer 20 may change, so that a quantity of ion-electron pairs formed in a contact section between the ionic gel layer 30 and the electrode layer 20 changes, thereby implementing force detection.

As shown in FIG. 14, the ionic gel layer 30 is in contact with the electrode layer 20, so that a thickness of the sensor 100 in the first direction Z can be further reduced, thereby facilitating a thinning design of the sensor 100.

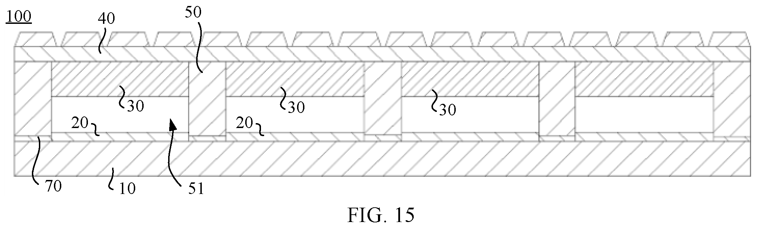

In the foregoing content, a microstructure 31 (as shown in FIG. 3 or FIG. 14) is disposed on a surface of the ionic gel layer 30 facing the electrode layer 20. However, the microstructure 31 may not be disposed on the surface of the ionic gel layer 30 facing the electrode layer 20. In other words, the surface of the ionic gel layer 30 facing the electrode layer 20 is a non-rough surface (or a plane), as shown in FIG. 15. FIG. 15 is a cross-sectional diagram of a seventh sensor according to an embodiment of this application.

It should be noted that, when no microstructure 31 is disposed on the surface of the ionic gel layer 30 facing the electrode layer 20, the ionic gel layer 30 and the electrode layer 20 should be disposed at intervals in the first direction Z, to ensure that the contact area between the ionic gel layer 30 and the electrode layer 20 may change when the sensor 100 is pressed.

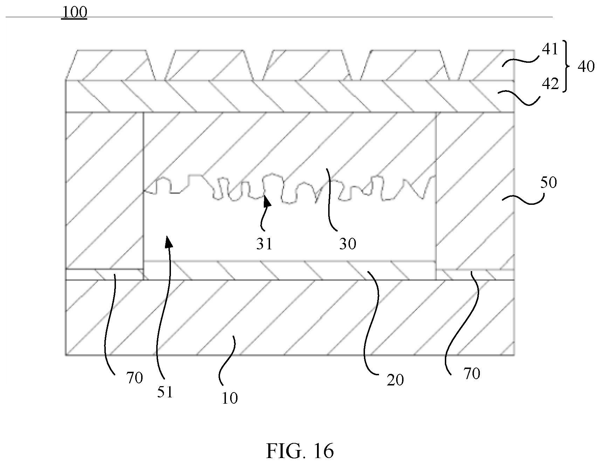

In the foregoing content, the support layer 50 has a plurality of through holes 51, so that the support layer 50 is of a grid structure. However, the support layer 50 may alternatively be provided with one through hole 51, as shown in FIG. 16. FIG. 16 is a cross-sectional diagram of an eighth sensor according to an embodiment of this application.

As shown in FIG. 16, the support layer 50 is provided with one through hole 51, and there are also one electrode layer 20 and one ionic gel layer 30. In addition, when there is one through hole 51, the support layer 50 is equivalent to one tube structure with two ends penetrated through.

An embodiment of this application further provides an ionic gel. The ionic gel includes an elastomer polymer skeleton and ionic liquid disposed in the elastomer polymer skeleton. The elastomer polymer skeleton includes 1,4-butanediol divinyl ether, 2,2'-(ethylenedioxy)diethanethiol, trimethylolpropane tris(3-mercaptopropionate), and benzoin dimethyl ether. The ionic liquid includes 1-ethyl-3-methylimidazolium bis(trifluoromethylsulfonyl)imide.

Specifically, the elastomer polymer skeleton is generated through click polymerization reaction by using 1,4-butanediol divinyl ether (BVB), 2,2'-(ethylenedioxy)diethanethiol (BMOE), and trimethylolpropane tris(3-mercaptopropionate) (TMPT) as monomers, and using benzoin dimethyl ether (DMPA) as a photoinitiator.

An embodiment of this application further provides a preparation method for an ionic gel, specifically including the following steps:
S1: Uniformly mix 2,2'-(ethylenedioxy)diethanethiol, trimethylolpropane tris(3-mercaptopropionate), benzoin dimethyl ether, 1,4-butanediol divinyl ether, and 1-ethyl-3-methylimidazolium bis(trifluoromethylsulfonyl)imide to obtain a precursor solution. Specifically, the precursor solution may be obtained by using the following steps.

S11: Mix the 2,2'-(ethylenedioxy)diethanethiol, the trimethylolpropane tris(3-mercaptopropionate), the benzoin dimethyl ether, and the 1-ethyl-3-methylimidazolium bis(trifluoromethylsulfonyl)imide until the benzoin dimethyl ether is completely dissolved and a liquid mixture is obtained.

To ensure complete dissolution of the benzoin dimethyl ether, an oscillation apparatus may be used to oscillate the liquid that is obtained by mixing the 2,2'-(ethylenedioxy)diethanethiol, the trimethylolpropane tris(3-mercaptopropionate), the benzoin dimethyl ether, and the 1-ethyl-3-methylimidazolium bis(trifluoromethylsulfonyl)imide.

During preparation of the liquid mixture, a ratio of 2,2'-(ethylenedioxy)diethanethiol, trimethylolpropane tris(3-mercaptopropionate), benzoin dimethyl ether, and 1-ethyl-3-methylimidazolium bis(trifluoromethylsulfonyl)imide is not limited herein. The ratio of 2,2'-(ethylenedioxy)diethanethiol, trimethylolpropane tris(3-mercaptopropionate), benzoin dimethyl ether, and 1-ethyl-3-methylimidazolium bis(trifluoromethylsulfonyl)imide is a mass ratio.

For example, the ratio of 2,2'-(ethylenedioxy)diethanethiol, trimethylolpropane tris(3-mercaptopropionate), benzoin dimethyl ether, and 1-ethyl-3-methylimidazolium bis(trifluoromethylsulfonyl)imide may be 93:22:20:1, which may further improve performance of the ionic gel. Certainly, the ratio of 2,2'-(ethylenedioxy)diethanethiol, trimethylolpropane tris(3-mercaptopropionate), benzoin dimethyl ether, and 1-ethyl-3-methylimidazolium bis(trifluoromethylsulfonyl)imide may alternatively be another ratio, for example, may alternatively be 92:22:20:1.

S12: Add the 1,4-butanediol divinyl ether to the liquid mixture to obtain the precursor solution.

Specifically, to fully mix the 1,4-butanediol divinyl ether and the liquid mixture, an oscillation apparatus may be used to perform oscillation for a specific time, and ultrasonic treatment is performed to remove bubbles. For example, after the 1,4-butanediol divinyl ether and the liquid mixture are mixed, oscillation is performed for 60s, and ultrasonic treatment is performed for 60s.

A proportion of the 1,4-butanediol divinyl ether is not limited herein. The proportion of the 1,4-butanediol divinyl ether is a mass ratio. For example, a ratio of 1,4-butanediol divinyl ether to benzoin dimethyl ether may be 102:1. Certainly, the ratio of 1,4-butanediol divinyl ether to benzoin dimethyl ether may alternatively be another ratio, for example, may alternatively be 101:1.

S2: Place the precursor solution under ultraviolet light to perform polymerization reaction, to obtain the ionic gel.

Specifically, the precursor solution may be irradiated by using an ultraviolet lamp, so that the precursor solution is cured to obtain the ionic gel.

In the descriptions of embodiments of this application, it should be noted that, unless otherwise clearly specified and limited, terms "mounted", "connected", and "connection" should be understood in a broad sense. For example, the term may be a fastened connection, may be an indirect connection through an intermediate medium, or may be an internal connection between two elements, or an interaction relationship between two elements. A person of ordinary skill in the art may understand specific meanings of the foregoing terms in embodiments of this application based on specific cases.

In embodiments of this application, it is implied that an apparatus or element in question needs to have a particular orientation, or needs to be constructed and operated in a particular orientation, and therefore cannot be construed as a limitation on embodiments of this application. In the descriptions of embodiments of this application, unless otherwise exactly and specifically ruled, "a plurality of" means two or more than two.

In the specification, claims, and accompanying drawings of embodiments of this application, the terms "first", "second", "third", "fourth", and the like (if existent) are intended to distinguish between similar objects but do not necessarily indicate a specific order or sequence. It may be understood that the data used in such a way is interchangeable in proper circumstances, so that embodiments of this application described herein can be implemented in other sequences than the sequence illustrated or described herein. In addition, the terms "include" and "have" and any other variants are intended to cover the non-exclusive inclusion. For example, a process, method, system, product, or device that includes a list of steps or units is not necessarily limited to those expressly listed steps or units, but may include other steps or units not expressly listed or inherent to such a process, method, product, or device.

The term "a plurality of" in this specification refers to two or more. The term "and/or" in this specification describes only an association relationship for describing associated objects and represents that three relationships may exist. For example, A and/or B may represent the following three cases: Only A exists, both A and B exist, and only B exists. In addition, a character "/" in this specification usually indicates an "or" relationship between associated objects, and a character "/" in a formula usually indicates a "divisible" relationship between associated objects.

It may be understood that various numbers in embodiments of this application are merely used for differentiation for ease of description, and are not used to limit the scope of embodiments of this application.

It should be understood that sequence numbers of the foregoing processes do not mean execution sequences in embodiments of this application. The execution sequences of the processes should be determined according to functions and internal logic of the processes, and should not be construed as any limitation on the implementation processes of embodiments of this application.

## Claims

1. A sensor, comprising a support layer, a substrate layer, an electrode layer, an ionic gel layer, and a packaging layer, wherein
the electrode layer is disposed between the ionic gel layer and the substrate layer, the ionic gel layer is disposed between the electrode layer and the packaging layer, and the packaging layer is connected to the substrate layer through the support layer; and
the sensor is configured to receive a pressing force, so that a contact area between the ionic gel layer and the electrode layer changes.

2. The sensor according to claim 1, wherein the ionic gel layer and the electrode layer are disposed at intervals in a first direction, and the first direction is a thickness direction of the substrate layer.

3. The sensor according to claim 1 or 2, wherein a microstructure is disposed on a surface of the ionic gel layer facing the electrode layer.

4. The sensor according to any one of claims 1 to 3, wherein the ionic gel layer comprises 1,4-butanediol divinyl ether, 2,2'-(ethylenedioxy)diethanethiol, trimethylolpropane tris(3-mercaptopropionate), benzoin dimethyl ether, and 1-ethyl-3-methylimidazolium bis(trifluoromethylsulfonyl)imide.

5. The sensor according to any one of claims 1 to 4, wherein the ionic gel layer is connected to the packaging layer through chemical bonding.

6. The sensor according to claim 5, wherein the sensor further comprises a coupling agent layer, the coupling agent layer is disposed between the ionic gel layer and the packaging layer, the ionic gel layer is connected to the coupling agent layer through chemical bonding, and the packaging layer is connected to the coupling agent layer through chemical bonding.

7. The sensor according to any one of claims 1 to 6, wherein the support layer is provided with a through hole, and the ionic gel layer and the electrode layer are disposed inside the through hole.

8. The sensor according to claim 7, wherein the support layer comprises a plurality of through holes disposed at intervals, and one ionic gel layer and one electrode layer are disposed in each of the through holes.

9. The sensor according to any one of claims 1 to 8, wherein the packaging layer comprises a first elastic layer and a second elastic layer, and the second elastic layer is disposed between the first elastic layer and the ionic gel layer and is bonded to the ionic gel layer; and
an elastic modulus of the first elastic layer is greater than or equal to 1 GPa and an elastic modulus of the second elastic layer is less than or equal to 100 MPa, or a ratio of the elastic modulus of the first elastic layer to the elastic modulus of the second elastic layer is greater than or equal to 10.

10. The sensor according to claim 9, wherein at least one of the first elastic layer and the second elastic layer is provided with a groove structure.

11. A detection apparatus, comprising a pressurization member and the sensor according to any one of claims 1 to 10, wherein
the pressurization member is configured to apply pressure to a measured site; and
the sensor is configured to measure a pulse wave signal at the measured site, and the sensor is disposed between the pressurization member and the measured site.

12. A wearable device, comprising a watch body, a first watch band, a second watch band, and the sensor according to any one of claims 1 to 10, wherein
two opposite ends of the watch body are respectively connected to one end of the first watch band and one end of the second watch band, and the sensor is connected to the first watch band.

13. An ionic gel, comprising an elastomer polymer skeleton and ionic liquid disposed in the elastomer polymer skeleton, wherein
the elastomer polymer skeleton comprises 1,4-butanediol divinyl ether, 2,2'-(ethylenedioxy)diethanethiol, trimethylolpropane tris(3-mercaptopropionate), and benzoin dimethyl ether; and
the ionic liquid comprises 1-ethyl-3-methylimidazolium bis(trifluoromethylsulfonyl)imide.

14. A preparation method for an ionic gel, comprising the following steps:
uniformly mixing 2,2'-(ethylenedioxy)diethanethiol, trimethylolpropane tris(3-mercaptopropionate), benzoin dimethyl ether, 1,4-butanediol divinyl ether, and 1-ethyl-3-methylimidazolium bis(trifluoromethylsulfonyl)imide to obtain a precursor solution; and
placing the precursor solution under ultraviolet light to perform polymerization reaction, to obtain the ionic gel.

15. The preparation method according to claim 14, wherein uniformly mixing the 2,2'-(ethylenedioxy)diethanethiol, the trimethylolpropane tris(3-mercaptopropionate), the benzoin dimethyl ether, the 1,4-butanediol divinyl ether, and the 1-ethyl-3-methylimidazolium bis(trifluoromethylsulfonyl)imide to obtain the precursor solution comprises the following steps:
mixing the 2,2'-(ethylenedioxy)diethanethiol, the trimethylolpropane tris(3-mercaptopropionate), the benzoin dimethyl ether, and the 1-ethyl-3-methylimidazolium bis(trifluoromethylsulfonyl)imide until the benzoin dimethyl ether is completely dissolved and a liquid mixture is obtained; and
adding the 1,4-butanediol divinyl ether to the liquid mixture to obtain the precursor solution.
